(19) Europäisches Patentamt / European Patent Office / Office européen des brevets

(11) **EP 4 483 816 B1**

(12) **EUROPEAN PATENT SPECIFICATION**

(45) Date of publication and mention
of the grant of the patent:
**21.01.2026 Bulletin 2026/04**

(21) Application number: **22933624.3**

(22) Date of filing: **21.11.2022**

(51) International Patent Classification (IPC):
**A61B 10/00** (2006.01)   **A61B 3/113** (2006.01)
**A61B 5/16** (2006.01)

(52) Cooperative Patent Classification (CPC):
**A61B 3/113; A61B 5/163; A61B 5/168**

(86) International application number:
**PCT/JP2022/042990**

(87) International publication number:
**WO 2023/181494 (28.09.2023 Gazette 2023/39)**

(54) **EVALUATION DEVICE AND EVALUATION PROGRAM**

AUSWERTUNGSVORRICHTUNG UND AUSWERTUNGSPROGRAMM

DISPOSITIF D'ÉVALUATION ET PROGRAMME D'ÉVALUATION

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **24.03.2022 JP 2022048808**

(43) Date of publication of application:
**01.01.2025 Bulletin 2025/01**

(73) Proprietor: **Sysmex Corporation
Kobe-shi, Hyogo 651-0073 (JP)**

(72) Inventors:
• **SAITO, Atsushi**
**Yokohama-shi, Kanagawa 221-0022 (JP)**
• **NINOMIYA, Masaru**
**Yokohama-shi, Kanagawa 221-0022 (JP)**
• **HAKOSHIMA, Shuji**
**Yokohama-shi, Kanagawa 221-0022 (JP)**
• **EBIHARA, Taeko**
**Yokohama-shi, Kanagawa 221-0022 (JP)**

(74) Representative: **Wimmer, Hubert
Wagner & Geyer Partnerschaft mbB
Patent- und Rechtsanwälte
Gewürzmühlstrasse 5
80538 München (DE)**

(56) References cited:
WO-A1-2014/208761     WO-A1-2021/039883
CN-A- 113 362 961     US-A1- 2017 027 805
US-A1- 2017 112 424     US-A1- 2017 188 930
US-A1- 2020 383 626     US-A1- 2021 290 133

• TSUCHIYA KENJI J., HAKOSHIMA SHUJI, HARA
TAKESHI, NINOMIYA MASARU, SAITO MANABU,
FUJIOKA TORU, KOSAKA HIROTAKA, HIRANO
YOSHIYUKI, : "Diagnosing Autism Spectrum
Disorder Without Expertise: A Pilot Study of 5- to
17-Year-Old Individuals Using Gazefinder",
FRONTIERS IN NEUROLOGY, vol. 11,
XP093092124, DOI: 10.3389/fneur.2020.603085

**Description**

Field

The present disclosure relates to an assessment device, an assessment method, and an assessment program.
Background

A method for assessing developmental disabilities, such as autism spectrum disorder (ASD), by detecting gaze points of a subject when images, such as human images and geometric patterns, are shown to the subject has been proposed (for example, JP 2017-99513 A).

US 2017 112424 A discloses a diagnosis supporting device which includes a display, an imaging unit configured to capture a subject, an eye gaze detector configured to detect an eye gaze direction of the subject from a captured image captured by the imaging unit, a gaze point detector configured to detect a gaze point of the subject in a display region of the display, based on the eye gaze direction, an output controller configured to display, on the display, a diagnosis image that includes a person image and a geometric pattern image, and in which at least one of contrast, color density, and a degree of transmittance of the person image or the geometric pattern image is changed over time, and an evaluator configured to calculate an evaluation value of the subject, based on the gaze point detected by the gaze point detector when the diagnosis image is displayed.

US 2017 188930 A discloses a systems and methods for quantitative assessment of autism spectrum disorder based on detected responses of a subject to displayed animations are disclosed.

US 2017 027805 A discloses a devices and methods for aiding in the diagnosis of and response to conditions ranging from unpleasant to disabling.

US 2021 290133 A discloses an evaluation device which includes a display configured to display evaluation purpose images; a gaze point detecting unit configured to detect positional data of a gaze point of a subject who observes the display; an area setting unit configured to set determination areas in the evaluation purpose images; a determination unit configured to determine, based on the positional data of the gaze point, whether the gaze point is present inside the determination areas to define determination values for the determination areas; an arithmetic unit configured to calculate, based on the determination values, a number of the at least one determination area in which the gaze point is present; and an evaluation unit configured to obtain evaluation data for evaluating a possibility that the subject is a person with a developmental disability based on the number of the at least one determination area in which the gaze point is present.

US 2020 383626 A discloses a cognitive impairment diagnostic apparatus which includes: a display which displays a video for diagnosis of cognitive impairment on a display surface; an imaging unit which captures images of an eye of a subject; a detection unit which detects viewpoints of the subject on the display surface in time series based on the images captured by the imaging unit; a creation unit which creates a distribution map representing a distribution of the viewpoints detected by the detection unit; a storage unit which stores case characteristic data indicating a characteristic of a viewpoint distribution corresponding to a typical case in cognitive impairment; and a diagnostic unit which diagnoses cognitive impairment of the subject by determining whether the distribution map has the characteristic indicated by the case characteristic data.

Summary

For the assessment technique described in JP 2017-99513 A, it is necessary to achieve a more accurate assessment of characteristics specific to a subject with ASD.

The present disclosure has been achieved in view of the above challenge, and aims to provide an assessment device, an assessment method, and an assessment program that enable to improve the accuracy in assessment of characteristics specific to a subject with ASD.

In accordance with the present invention, an assessment device and an assessment program as set forth in the appended claims is provided. In particular, an assessment device according to the present disclosure includes: a display unit; a gaze-point detecting unit configured to detect a position of a gaze point of a subject; a display control unit configured to display a plurality of unit images including at least one of an object having specificity and an object having sensitivity on the display unit in different tasks; a region setting unit configured to set, for each of the tasks, an object determination region corresponding to each of the objects and an entire determination region corresponding to an entirety of the display unit; a determining unit configured to determine whether the gaze point is present in each of the object determination region and the entire determination region based on the detected position of the gaze point; an arithmetic unit configured to calculate an object gaze rate indicating a rate at which the gaze point is present in the object determination region in each of the tasks, and an entire gaze rate indicating a rate at which the gaze point is present in the entire determination region throughout all the tasks based on a determination result of the determining unit; and an assessing unit configured to calculate a score based on the object gaze rate and the entire gaze rate calculated by the arithmetic unit, and acquire assessment data based on the calculated score.

An assessment program according to the present disclosure causes a computer to execute the processes of: detecting a position of a gaze point of a subject; displaying a plurality of unit images including at least one of an object having specificity and an object having sensitivity on a display unit in different tasks; setting an object determination region corresponding to each of the objects and an entire determination region corresponding to an entirety of the display unit; determining whether the gaze point is present in each of the object determination region and the entire determination region based on the detected position of the gaze point; calculating an object gaze rate indicating a rate at which the gaze point is present in the object determination region in each of the tasks, and an entire gaze rate indicating a rate at which the gaze point is present in the entire determination region throughout all the tasks based on a determination result; and calculating a score based on the object gaze rate and the entire gaze rate calculated, to acquire assessment data based on the calculated score.

According to the present disclosure, it is possible to improve the accuracy of assessment of characteristics specific to a subject with ASD.

Brief Description of Drawings

   FIG. 1 is a diagram schematically illustrating an example of an assessment device according to the present embodiment.
   FIG. 2 is a functional block diagram illustrating an example of the assessment device.
   FIG. 3 is a diagram illustrating an example of a unit image displayed on a display unit.
   FIG. 4 is a flowchart illustrating an example of an assessment method according to the present embodiment.
   Description of Embodiments

Hereinafter, embodiments of an assessment device, an assessment method, and an assessment program according to the present disclosure will be explained based on the drawings. Note that the embodiments are not intended to limit the present invention. Moreover, components in the embodiments described below include those easily replaceable by a person skilled in the art, or those substantially identical.

Assessment Device

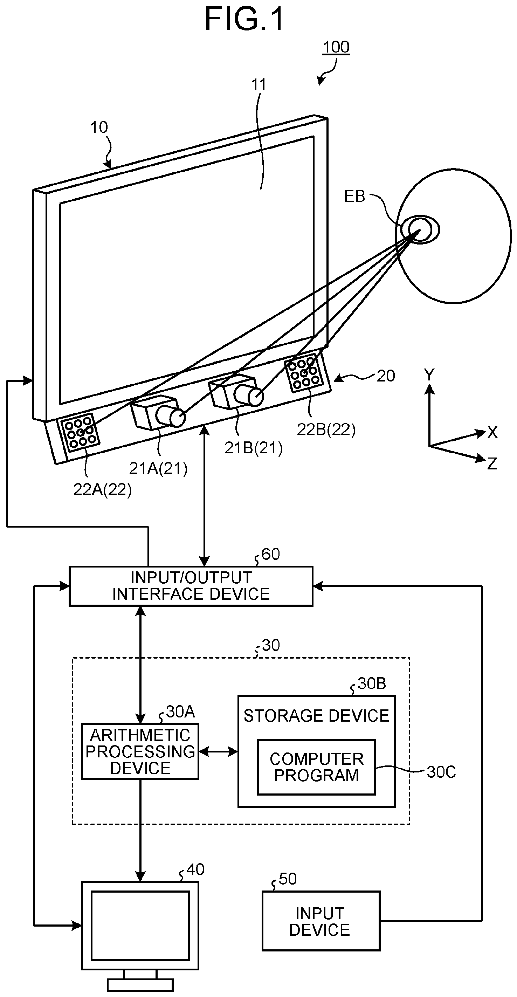

FIG. 1 is a diagram schematically illustrating an example of an assessment device 100 according to the present embodiment. The assessment device 100 according to the present embodiment detects a line of sight of a subject, and performs assessment of autism spectrum disorder (hereinafter, denoted as "ASD") by using detection results. The assessment device 100 can detect a line of sight of a subject by various methods, such as a method of detecting a line of sight based on a position of the pupil of the subject and a position of the corneal reflection image, or a method of detecting a line of sight based on a position of the inner canthus and a position of the iris of the subject.

As illustrated in FIG. 1, the assessment device 100 includes a display device 10, an image acquiring device 20, a computer system 30, an output device 40, an input device 50, and an input/output interface device 60. The display device 10, the image acquiring device 20, the computer system 30, the output device 40, and the input device 50 performs data communication through the input/output interface device 60. The display device 10 and the image acquiring device 20 respectively have driving circuits not illustrated.

The display device 10 includes a flat panel display, such as a liquid crystal display (LCD) or an organic electroluminescence display (OLED). In the present embodiment, the display device 10 includes the display unit 11. The display unit 11 displays information, such as an image. The display unit 11 is substantially parallel to an XY plane. An X axis direction on the XY plane is a left and right direction of the display unit 11, and a Y axis direction is an up and down direction of the display unit 11, and a Z axis direction is a depth direction perpendicular to the display unit 11. The display device 10 may be a head-mounted display device. When the display device 10 is a head-mounted display device, a component, such as the image acquiring device 20, is to be arranged in a head-mounted module.

The image acquiring device 20 acquires image data of left and right eyeballs EB of a subject, and transmits the acquired image data to the computer system 30. The image acquiring device 20 includes an imaging device 21. The imaging device 21 captures the left and right eyeballs EB of the subject to acquire image data. The imaging device 21 has various kinds of cameras according to methods of detecting a line of sight of a subject. For example, in the case of a method of detecting a line of sight based on a position of the pupil of the subject and a position of the corneal reflection image, the imaging device 21 has an infrared camera, and includes an optical system that is capable of transmitting near-infrared light of, for example, a wavelength of 850 nm, and a imaging device that is capable of receiving the near-infrared light. Moreover, in the case of a method of detecting a line of sight based on a position of the inner canthus and a position of the iris of a subject, the imaging device 21 has a visible light camera. The imaging device 21 outputs a frame synchronization signal.

The period of the frame synchronization signal can be set to, for example, 20 milliseconds (msec), but is not limited thereto. The imaging device 21 can be configured, for example, as a stereo camera having a first camera 21A and a second camera 21B, but it is not limited thereto.

Furthermore, for example, in the case of the method of detecting a line of sight based on a position of the pupil of a subject and a position of the corneal reflection image, the image acquiring device 20 includes an illumination device 22 that illuminates the eyeballs EB of the subject. The illumination device 22 includes a light emitting diode (LED) light source, and is capable of emitting near-infrared light of, for example, a wavelength of 850 nm. In the case of the method of detecting a line of sight based on a position of the inner canthus and a position of the iris of a subject, the illumination device 22 is not necessary to be arranged. The illumination device 22 emits detection light so as to synchronize with the frame synchronization signal with the imaging device 21. The illumination device 22 can be configured to have, for example, a first light source 22A and a second light source 22B, but it is not limited thereto.

The computer system 30 comprehensively controls the operation of the assessment device 100. The computer system 30 includes an arithmetic processing device 30A and a storage device 30B. The arithmetic processing device 30A includes a micro-processor such as a central processing unit (CPU). The storage device 30B includes a memory, such as a read only memory (ROM) and a random access memory (RAM), or a storage. The arithmetic processing device 30A performs arithmetic processing according to a computer program 30C stored in the storage device 30B.

The output device 40 includes a display device such as a flat panel display. The output device 40 may include a printing device. The input device 50 is operated to generate input data. The input device 50 includes a keyboard or a mouse for a computer system. When the input device 50 may include a touch sensor that is arranged on a display unit of the output device 40 being a display device.

The assessment device 100 according to the present embodiment has the display device 10 and the computer system 30 as separate devices. The display device 10 and the computer system 30 maybe integrated. For example, the assessment device 100 may include a tablet-type personal computer. In this case, the display device, the image acquiring device, the computer system, the input device, the output device, and the like may be mounted on the tablet-type personal computer.

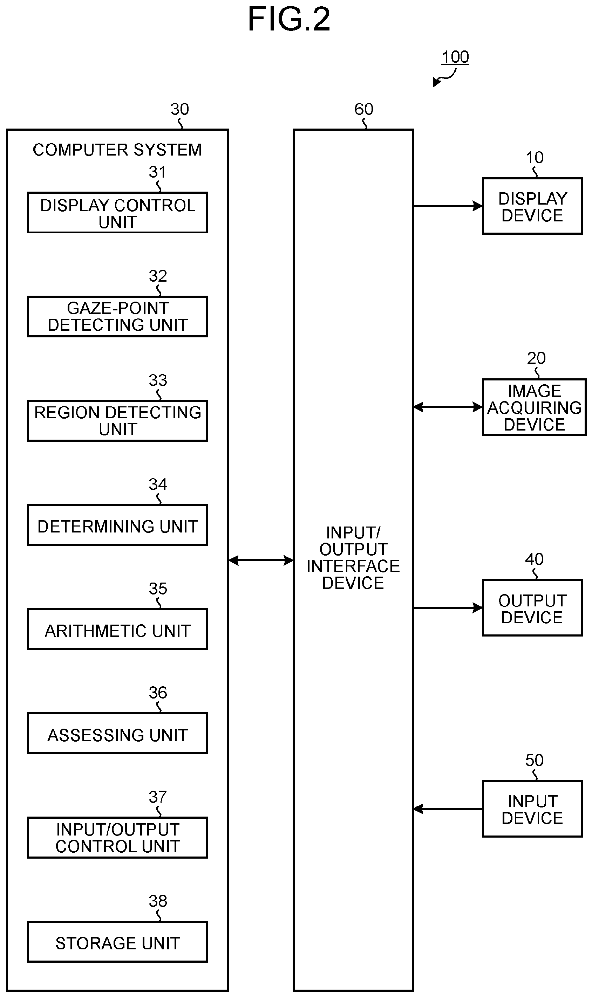

FIG. 2 is a functional block diagram illustrating an example of the assessment device 100. As illustrated in FIG. 2, the computer system 30 includes a display control unit 31, a gaze-point detecting unit 32, a region setting unit 33, a determining unit 34, an arithmetic unit 35, an assessing unit 36, an input/output control unit 37, and a storage unit 38. The functions of the computer system 30 are implemented by the arithmetic processing device 30A and the storage device 30B (refer to FIG. 1). The computer system 30 may be configured such that a part of the functions is arranged outside the assessment device 100.

The display control unit 31 displays an assessment image on the display unit 11. In the present embodiment, the assessment image includes multiple unit images including at least one of an object with specificity, such as a person and an item (hereinafter, denoted as "specificity object") and an object with sensitivity, such as a figure and a geometric pattern (hereinafter, denoted as "sensitivity object"). The unit image includes at least one of a still image and a moving image. The specificity object is an object that tends to be gazed by a subject having low possibility of having ASD. Examples of the specificity object include a person, an item, and the like. The sensitivity object is an object that tends to be gazed by a subject having high possibility of having ASD. Examples of the sensitivity object include a figure, a geometric pattern, and the like.

In the assessment image, the specificity object and the sensitivity object may be present within the same unit image, or may be present in different unit images. The display control unit 31 displays a single unit image on the display unit 11 as a single task as the assessment image. The display control unit 31 displays multiple pre-prepared unit images on the display unit 11 in different tasks. The display control unit 31 can display the assessment image described above on the display unit 11, for example, as an assessment moving image, but the display mode is not limited to the assessment moving image, and it may be a still image.

The gaze-point detecting unit 32 detects position data of a gaze point of a subject. In the present embodiment, the gaze-point detecting unit 32 detects a line-of-sight vector of a subject that is defined by three-dimensional global coordinate system based on image data of the left and right eyeball EB of the subject acquired by the image acquiring unit 20. The gaze-point detecting unit 32 detects position data of an intersection of the detected line-of-sight vector of the subject and the display unit 11 of the display device 10 as the position data of the gaze point of the subject. That is, in the present embodiment, the position data of the gaze point is position data of an intersection of a line-of-sight vector of a subject defined by the three-dimensional global coordinate system and the display unit 11 of the display device 10. The gaze-point detecting unit 32 detects the position data of a gaze point of a subject at each sampling period. This sampling period may be, for example, the period (for example, each 20 msec) of the frame synchronization signal output from the imaging device 21.

The region setting unit 33 sets, in the display unit 11, an object determination region corresponding to respective objects and an entire determination region corresponding to the entirety of the display unit 11 for each task. In the present embodiment, the respective determination regions set by the region setting unit 33 are basically not displayed on the display unit 11. However, the respective determination regions may be configured to be displayed by a control of the display control unit 31. The object determination region is included in the entire determination region.

The determining unit 34 respectively determines whether a gaze point is present in a determination region based on the position data of the gaze point, and outputs the determination result as determination data. The determining unit 34 determines whether a gaze point is present in a determination region at each determination period. The determination period may be, for example, the period (for example, each 20 msec) of the frame synchronization signal output from the imaging device 21. That is, the determination period of the determining unit 34 is the same as the sampling period of the gaze-point detecting unit 32. The determining unit 34 performs, each time a position of a gaze point is sampled by the gaze-point detecting unit 32, determination about the gaze point, and outputs determination data. When multiple determination regions are set, the determining unit 34 can determine whether a gaze point is present for each of the determination regions, to output the determination data.

The arithmetic unit 35 calculates an object gaze rate and an entire gaze rate based on the determination result of the determining unit 34. The object gaze rate indicates a rate at which a gaze point is present in the object determination region in each task. The entire gaze rate indicates a rate at which a gaze point is present in the entire determination region throughout all tasks. The arithmetic unit 35 includes a timer to detect elapsed time since the assessment image is displayed on the display unit 11, and a counter that counts the number of times of determination that a gaze point is present in the object determination region and the entire determination region by the determining unit 34.

The arithmetic unit 35 calculates present time data of a gaze point based on the determination data of the determining unit 34. The present time data is data indicating time in which a gaze point is present in the determination region. As the number of times of determination that a gaze point is present in the determination region by the determining unit 34 increases, it is possible to estimate that presence time during which the gaze point is present in the determination region is longer. Therefore, presence time data can be regarded as the number of times of determination that a gaze point is present in the determination region by the determining unit 34.

The arithmetic unit 35 calculates the object gaze rate that indicates a rate at which the gaze point is present in the object determination region in each task based on, for example, time from start of one task of the assessment image to completion, and the presence time data in the object determination region. Moreover, the arithmetic unit 35 calculates the entire gaze rate that indicates a rate at which the gaze point is present in the entire determination region throughout all tasks based on time from start of the first task of the assessment image to completion of the last task, and the presence time data in the entire determination region.

The assessing unit 36 calculates a score to assess a subject based on the object gaze rate and the entire gaze rate. The assessing unit 36 acquires assessment data of the subject based on the calculated score. At calculation of the score, the assessing unit 36 may calculate a score, assigning a heavier weight to the entire gaze rate than the object gaze rate. Furthermore, the assessing unit 36 may calculate a correlation coefficient indicating a strength of correlation between reference data stored in the storage unit 38 described later and the object gaze rate and the entire gaze rate calculated by the arithmetic unit 35, and may calculate the score, setting weights to the object gaze rate and the entire gaze rage based on the calculated correlation coefficient.

The input/output control unit 37 acquires data (image data of the eyeballs EB, input data, and the like) from at least one of the image acquiring unit 20 and the input device 50. Moreover, the input/output control unit 37 outputs data to at least one of the display device 10 and the output device 40. The input/output control unit 37 may output a problem for a subject from the output device 40, such as a speaker.

The storage unit 38 stores various kinds of data, such as the determination data, the presence time data, the reference data of the object gaze rate and the entire gaze rate, and the assessment data described above. The reference data of the object gaze rate and the entire gaze rate is data including a calculation result of the object gaze rate and the entire gaze rate of a subject diagnosed as ASD, and a calculation result of the object gaze rate and the entire gaze rate of a subject diagnosed as not having ASD.

Moreover, the storage unit 38 stores an assessment program that causes a computer to execute processing of detecting a position of a gaze point of a subject; processing of displaying multiple different images including an object corresponding to at least one of the specificity region and the sensitivity region on the display unit in different tasks; processing of setting, for each task, the object determination region corresponding to respective objects and the entire determination region corresponding to the entirety of the display unit; processing of determining whether a gaze point is present in each of the object determination region and the entire determination region for each task, based on the detected position of the gaze point; processing of calculating the object gaze rate that indicates a rate at which a gaze point is present in the object determination region in each task and the entire gaze rate that indicates a rate at which a gaze point is present in the entire determination region throughout all tasks based on the determination result of the determining unit; and processing of calculating a score based on the object gaze rate and the entire gaze rate calculated by the arithmetic unit, and of acquiring the assessment data based on the calculated score.

Assessment Method

Next, an assessment method according to the present embodiment will be explained. In the assessment method according to the present embodiment, whether the assessment data of a subject exceeds a predetermined threshold is assessed by using the assessment device 100 described above.

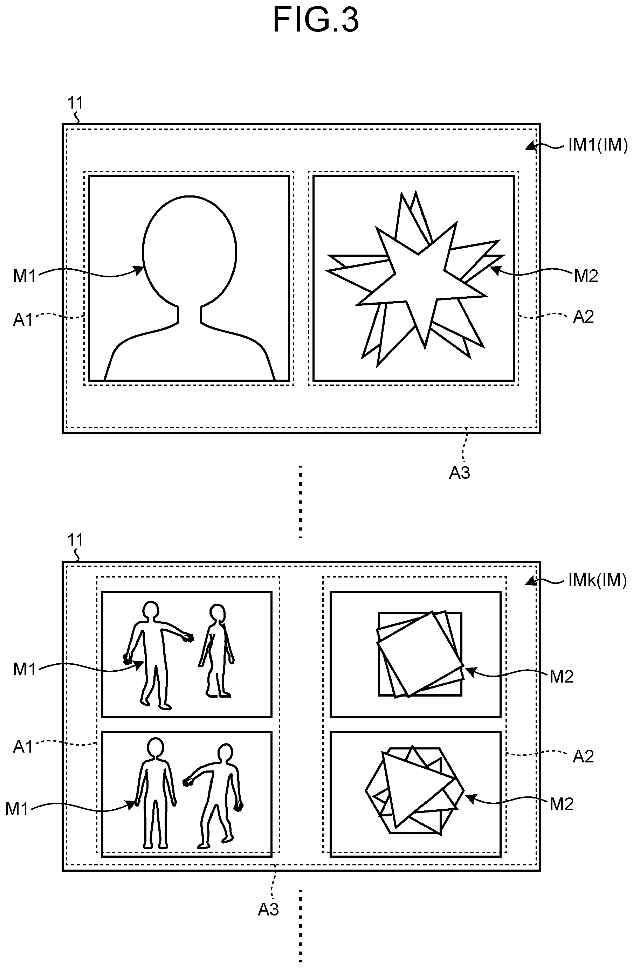

FIG. 3 is a diagram illustrating an example of the unit image displayed on the display unit 11. As illustrated in FIG. 3, the display control unit 31 displays a unit image IM including at least one of a specificity object M1 and a sensitivity object M2 in a single task. In the example illustrated in FIG. 3, the specificity object M1 is, for example, a person, and the sensitivity object M2 is, for example, a figure. The display control unit 31 displays a different unit image IM on the display unit 11 in each task. In FIG. 3, the unit image IM displayed in the first task is denoted as unit image IM1, and the unit image displayed in the k-th task is denoted as unit image IMk. The display control unit 31 may display an image including only one of the specificity object M1 and the sensitivity object M2 on the display unit 11. In this case, it is possible to prevent the interest of the subject from being dispersed.

The region setting unit 33 sets object determination regions A1, A2 corresponding to the specificity object M1 and the sensitivity object M2. The region setting unit 33 sets the object determination regions A1, A2 in ranges not overlapping each other. The object determination regions A1, A2 are not displayed on the display unit 11. Moreover, the region setting unit 33 sets an entire determination region A3 corresponding to the entirety of the display unit 11. The object determination regions A1, A2 are a part of the entire determination region A3, and are included in the entire determination region A3.

When setting the object determination regions A1, A2, the region setting unit 33 can be configured to set them within two regions in a single task. For example, when the specificity object M1 and the sensitivity object M3 are included in the single unit image IM displayed in a single task, it may be set as one region for the specificity object M1 and one region for the sensitivity object M2. For example, when the specificity object M1 and the sensitivity object M2 are included in the single unit image IM, the region setting unit 33 may set as one region for the multiple specificity objects M1 together, and one region for the multiple sensitivity objects M2 together. The shapes of the object determination regions A1, A2 may be, for example, a rectangular shape, a circular shape, an elliptical shape, a polygonal shape, and the like, and are not limited. The sizes of the multiple object determination regions A1, A2 may be different.

Generally, subjects with a high possibility of having ASD tend to have a stronger interest (fixation) in the sensitivity object such as a figure and a geometric pattern than the specificity object, such as a person, an animal, and a plant, compared to subjects with a low possibility of having ASD. In addition to this fact, the inventors of the present invention have discovered that the subjects with a high possibility of having ASD tend to have a lower percentage of gazing at the display unit 11 throughout all tasks compared to the subjects with a low possibility of having ASD. Accordingly, in the present embodiment, assessment is performed by detecting a gaze point of a subject after instructing to look at an assessment image on the display unit 11 to the subject, and by acquiring the assessment data based on the detection result.

First, the display control unit 31 displays the assessment image on the display unit 11. The assessment image includes multiple tasks in chronological order. In a single task, a single unit image IM is displayed on the display unit 11. The display control unit 31 may display an eye-catch image to move a gaze point to a desired position on the display unit 11 at the beginning of one task. Moreover, the region setting unit 33 sets the object determination region A1 corresponding to the specificity object M1, the object determination region A2 corresponding to the sensitivity object M2, and the entire determination region A3 corresponding to the entirety of the display unit 11 in a state in which the unit image IM of the assessment image is displayed.

After one task is finished, the display control unit 31 displays another unit image on the display unit 11 in a next task. The region setting unit 33 sets the object determination regions A1, A2, and the entire determination region A3 in a state in which the other unit image is displayed. As described, a different unit image is displayed on the display unit 11 by the display control unit 31 in each task. Moreover, the region setting unit 33 sets the object determination regions A1, A2, and the entire determination region A3 corresponding to the unit image in each task. The display control unit 31 may set a task of displaying a unit image that is not used for detection of the object gaze rate on the display unit 11.

The gaze-point detecting unit 32 detects a position of a gaze point of a subject at every defined sampling period (for example, 20 msec) in a period in which the assessment image is displayed. When a position of a gaze point of a subject is detected, the determining unit 34 determines whether the gaze point is present in the object determination regions A1, A2, and the entire determination region A3, and outputs the determination data. Therefore, the determining unit 34 outputs the determination data every determination period same as the sampling period described above each time a position of a gaze point is sampled by the gaze-point detecting unit 32.

The arithmetic unit 35 calculates gaze point data of the subject based on the determination data. The arithmetic unit 35 calculates the presence time data as gaze point data. The presence time data indicates presence time in which a gaze point of the subject is present in the object determination regions A1, A2, and the entire determination region A3. In the present embodiment, as the number of times of determination that a gaze point is present in the object determination regions A1, A2, and the entire determination region A3 by the determining unit 34 increases, it is possible to estimate that the presence time in which a gaze point is present in the object determination regions A1, A2, and in the entire determination region A3 is longer. Therefore, the presence time data can be regarded as the number of times of determination that a gaze point is present in the object determination regions A1, A2, and in the entire determination region A3 by the determining unit 34.

The arithmetic unit 35 calculates the object gaze rate of the specificity object M1 and the object gaze rate of the sensitivity object M2 based on the presence time data of the object determination region A1 and the object determination region A2 calculated for one task and time from start to completion of the one task. The arithmetic unit 35 calculates the object gaze rate for each task. The arithmetic unit 35 calculates the object gaze rate in one task for three kinds of periods of, for example, the first 1 second of the task, the first 2 seconds, and the entire duration of the task. The arithmetic unit 35 can use a value showing a correlation with the reference data described later out of the calculated three kinds of object gaze rates. Moreover, when an eye-catch image is displayed at the beginning of a task by the display control unit 31, the period in which the eye-catch image is displayed can be excluded from the time range for calculation of the object gaze rate. For example, when the first 0.3 seconds of the task is the display period of the eye-catch image, the arithmetic unit 35 can calculate the object gaze rate starting from 0.3 seconds after the beginning of the task.

Furthermore, the arithmetic unit 35 calculates the entire gaze rate in the entirety of the display unit 11 based on the presence time data of the entire determination region A3 calculated in the respective tasks and time from start of the first task to completion of the last task.

The assessing unit 36 acquires an assessment value based on the calculated object gaze rate and entire gaze rate, and acquires the assessment data based on the assessment value. In the present embodiment, when the object gaze rate of the specificity object M1 is R1, the object gaze rate of the sensitivity object M2 is R2, and the entire gaze rate is R3, an assessment score TS can be expresses as

$$TS = \sum_k \left( \sum_m C1_{k,\ m} \cdot R1_{k,\ m} + \sum_n C2_{k,\ n} \cdot R2_{k,\ n} \right) + C3 \cdot R3$$

where C1 represents a coefficient indicating a weight of the object gaze rate R1 of the specificity object M1, C2 represents a coefficient indicating a weight of the object gaze rate R2 of the sensitivity object M2, and C3 represents a coefficient indicating a weight of the entire gaze rate R3. k represents a number of task. m represents a number of a specificity region. n represents a number of a sensitivity region.

Subjects having a low possibility of having ASD have a tendency that the object gaze rate R1 of the specificity object M1 is high and the object gaze rate R2 of the sensitivity object M2 is low, compared to subjects having a high possibility of having ASD. That is, the subjects having a low possibility of having ASD tend to have a higher value for the value indicating specificity and a lower value for the value indicating sensitivity. On the other hand, subjects having a high possibility of having ASD have a tendency that the object gaze rate R2 of the sensitivity object M2 is high and the object gaze rate R1 of the specificity object M1 is low, compared to subjects having a low possibility of having ASD. That is, the subjects having a high possibility of having ASD have a higher value for the value indicating sensitivity and a lower value of the value indicating specificity.

Subjects having a lower possibility of having ASD have a tendency that the entire gaze rate R3 is high compared to subjects having a higher possibility of having ASD. On the other hand, subjects having a higher possibility of having ASD have a tendency that the entire gaze rate R3 is low compared to subjects having a lower possibility of having ASD. From these results, the entire gaze rate R3 can be used as a value indicating a tendency of specificity.

The assessing unit 36 can set values of C1, C2, and C3 such that the weight of the entire gaze rate R3 is heavier than that of the object gaze rates R1, R2. Moreover, the assessing unit 36 calculates a correlation coefficient indicating a strength of correlation between the reference data stored in the storage unit 38 and the object gaze rates R1, R2 calculated by the arithmetic unit 35, and can set the C1, C2 based on the calculated correlation coefficient.

In the present embodiment, the reference data is, for example, data of the object gaze rate and the entire gaze rate calculated in advance by making subjects diagnosed as having a low possibility of having ASD and subjects diagnosed as having a high possibility of having ASD gaze at respective unit images included in the assessment image. The assessing unit 36 can calculate a correlation coefficient based on a reference value and a calculation value of the object gaze rate of a subject to be assessed, using 0 as the reference value indicating the object gaze rate of the subjects diagnosed as having a low possibility of having ASD, and 1 as the reference value indicating the object gaze rate of the subjects diagnosed as having a high possibility of having ASD out of the reference data. As the reference value, an average value of the object gaze rate of multiple subjects or the like can be used. For example, when the correlation coefficient is lower than 0.25, the assessing unit 36 can set the coefficient of relevant C1, C2 to 0. Moreover, for example, when the correlation coefficient is 0.25 or higher, the assessing unit 36 can set the coefficient of relevant C1, C2 to 1. The method of setting the coefficients C1, C2 is not limited to the above and, for example, a value of the correlation coefficient may be used as the values of the coefficient of C1, C2.

The assessing unit 36 can set weights with opposite signs between the coefficients C1, C3 and the coefficient C2 such as setting a negative value to the coefficient C1 of the object gaze rate R1 and the entire gaze rate R3 that indicate a strength of specificity, and setting a positive value to the coefficient C2 of the object gaze rate R2 that indicates a strength of sensitivity. In this case, the smaller assessment score TS indicates the stronger specificity, and the larger assessment score TS indicates the stronger sensitivity. The assessing unit 36 can set, for example, a threshold to the assessment score TS, and can assess that specificity is strong when the assessment score TS is lower than the threshold, and assess that sensitivity is strong when the assessment score TS is equal to or higher than the threshold.

In the present embodiment, when the assessing unit 36 outputs the assessment data, the input/output control unit 37 can output, for example, text data of "the subject is considered to have strong specificity" or text data indicating "the subject is considered to have strong sensitivity" or the like according to the assessment data to the output device 40.

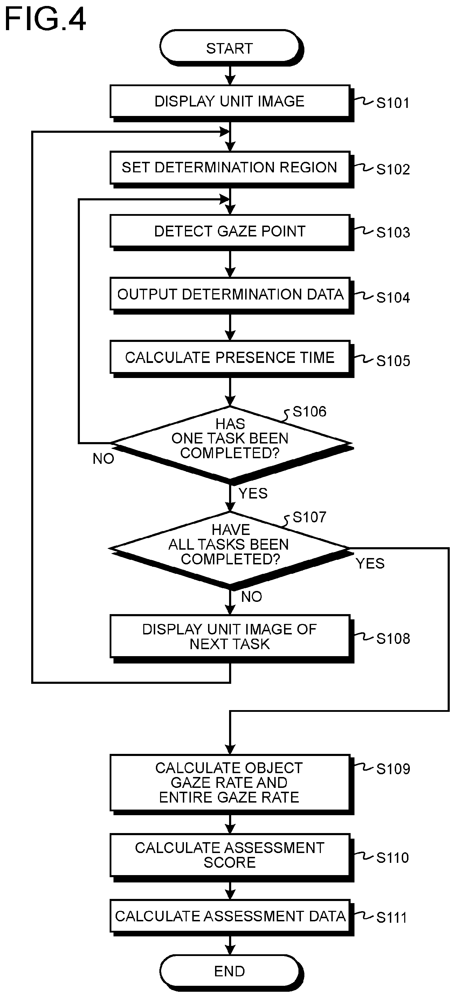

Next, an example of the assessment method according to the present embodiment will be explained, referring to FIG. 4. FIG. 4 is a flowchart illustrating an example of the assessment method according to the present embodiment. In the assessment method according to the present embodiment, as illustrated in FIG. 4, first, the display control unit 31 displays the unit image IM according to the first task as the assessment image on the display unit 11 (step S101). The region setting unit 33 sets the object determination regions A1, A2 corresponding to the specificity object M1 and the sensitivity object M2, and the entire determination region A3 corresponding to the entirety of the display unit 11 in the unit image IM (step S102).

Thereafter, the gaze-point detecting unit 32 samples positions of a gaze point at a predetermined sampling period to detect a position of a gaze point (step S103). The determining unit 34 determines whether a gaze point is present in the object determination regions A1, A2 and the entire determination region A3 each time a position of a gaze point is sampled by the gaze-point detecting unit 32 based on the position of the gaze point, and outputs the determination data (step S104). The arithmetic unit 35 calculates presence time of the object determination regions A1, A2 and the entire determination region A3 based on the determination data (step S105). The arithmetic unit 35 determines whether the display time in the task has ended (step S106). When it is determined that the display time has not ended (step S106: NO), the processing at step S103 and later is repeated.

When it is determined that the display time has ended (step S106: YES), the display control unit 31 determines whether all tasks have been finished (step S107). When it is determined that all tasks have not been finished (step S107: NO), the display control unit 31 causes the display unit 11 to display the unit image IM relating to the next task (step S108), and causes the processing at step S102 and later to be repeated.

When it is determined that all tasks have been finished (step S107: YES), the arithmetic unit 35 calculates the object gaze rate and the entire gaze rate based on the arithmetic results (step S109). The assessing unit 36 calculates the assessment score based on the calculation result of the arithmetic unit 35 (step S110), calculates the assessment data based on the calculated assessment score (step S111), and ends the processing.

As described above, the assessment device 100 according to the present embodiment includes the display unit 11, the gaze-point detecting unit 32 that detects a position of a gaze point of a subject, the display control unit 31 that displays multiple unit images including at least one of an object having specificity and an object having sensitivity on the display unit 11 in different tasks, the region setting unit 33 that sets the object determination region corresponding to each object and the entire determination region corresponding to the entirety of the display unit 11 in each task, the determining unit 34 that determines whether a gaze point is present in each of the object determination region and the entire determination region based on the detected position of the gaze point, the arithmetic unit 35 that calculates the object gaze rate indicating a rate at which a gaze point is present in the object determination region in each task and the entire gaze rate indicating a rate at which a gaze point is present in the entire determination region throughout all tasks based on the determination result of the determining unit 34, and the assessing unit 36 that calculates a score based on the object gaze rate and the entire gaze rate calculated by the arithmetic unit 35 to acquire the assessment data based on the calculated score.

The assessment method according to the present embodiment includes detecting a position of a gaze point of a subject; displaying multiple unit images including at least one of an object having specificity and an object having sensitivity on the display unit 11 in different tasks; setting the object determination region corresponding to each object and the entire determination region corresponding to the entirety of the display unit 11 for each task; determining whether a gaze point is present in each of the object determination region and the entire determination region based on the detected position of the gaze point; calculating the object gaze rate indicating a rate at which a gaze point is present in the object determination region in each task and the entire gaze rate at which a gaze point is present in the entire determination region throughout all tasks based on the determination result; and calculating a score based on the calculated object gaze rate and entire gaze rate, to acquire the assessment data indicating whether the calculated score exceeds a threshold.

The assessment program according to the present embodiment causes a computer to execute processing of detecting a position of a gaze point of a subject; processing of displaying multiple unit images including at least one of an object having specificity and an object having sensitivity on the display unit 11 in different tasks; processing of setting the object determination region corresponding to each object and the entire determination region corresponding to the entirety of the display unit 11 for each task; processing of determining whether a gaze point is present in the object determination region and the entire determination region based on the detected position of the gaze point; processing of calculating the object gaze rate indicating a rate at which a gaze point is present in the object determination region in each task and the entire gaze rate at which a gaze point is present in the entire determination region throughout all tasks based on the determination result; and processing of calculating a score based on the calculated object gaze rate and entire gaze rate, to acquire the assessment data based on the calculated score.

With this configuration, by calculating the object gaze rate, it is possible to assess the tendency of a subject exhibiting characteristics of either specificity or sensitivity. Furthermore, the inventors of the present invention have discovered that the subjects with a high possibility of having ASD tend to have a lower percentage of gazing at the display unit 11 throughout all tasks compared to the subjects with a low possibility of having ASD. Accordingly, by calculating the entire gaze rate of a subject in addition to the object gaze rate, it is possible to improve the accuracy of assessment of characteristics specific to subjects with ASD.

In the assessment device 100 according to the present embodiment, the assessing unit 36 calculates a score, assigning a heavier weight to the entire gaze rate than the object gaze rate. With this configuration, the assessment of the entire gaze rate of a subject can be reflected more strongly. Therefore, it is possible to assess characteristics specific to subjects with ASD more accurately.

The assessment device 100 according to the present embodiment further includes the storage unit 38 that stores the reference data of the object gaze rate and the entire gaze rate, and the assessing unit 36 calculates a correlation coefficient indicating a strength of correlation between the reference data stored in the storage unit 38 and the object gaze rate and the entire gaze rate calculated by the arithmetic unit, and assigns weights to the object gaze rate and the entire gaze rate based on the calculated correlation coefficient, to calculate a score. With this configuration, because weights are assigned to the object gaze rate and the entire gaze rate based on the reference data, characteristics specific to subjects with ASD can be assessed more accurately.

The technical scope of the present disclosure is not limited to the above embodiments, and various modifications can be made without departing from the gist of the disclosure.

The present disclosure contributes to the realization of the goal of SDGs, "Good Health and Well-being for All", and includes matters that contribute to value creation through healthcare products and services.

Industrial Applicability

The assessment device, the assessment method, and the assessment program according to the present disclosure can be used, for example, for processing device, such as a computer.

Reference Signs List

| | |
|---|---|
| A1, A2 | Object determination region |
| A3 | Entire determination region |
| M1 | Specificity object |
| M2 | Sensitivity object |
| EB | Eyeball |
| IM | Unit image |
| 10 | Display device |
| 11 | Display unit |
| 20 | Image acquiring device |
| 21 | Imaging device |
| 21A | First camera |
| 21B | Second camera |
| 22 | Illumination device |
| 22A | First light source |
| 22B | Second light source |
| 30 | Computer system |
| 30A | Arithmetic processing device |
| 30B | Storage device |
| 30C | Computer program |
| 31 | Display control unit |
| 32 | Gaze-point detecting unit |
| 33 | Region setting unit |
| 34 | Determining unit |

| 35 | Arithmetic unit |
|---|---|
| 36 | Assessing unit |
| 37 | Input/output control unit |
| 38 | Storage unit |
| 40 | Output device |
| 50 | Input device |
| 60 | Input/output interface device |
| 100 | Assessment device |

**Claims**

1. An assessment device (100) comprising:

   a display unit (11);
   a gaze-point detecting unit (32) configured to detect a position of a gaze point of a subject;
   a display control unit (31) configured to display a plurality of unit images including at least one of an object having specificity (M1) and an object having sensitivity (M2) on the display unit (11) in different tasks;
   a region setting unit (33) configured to set, for each of the tasks, an object determination region (A1, A2) corresponding to each of the objects and an entire determination region (A3) corresponding to an entirety of the display unit (11);
   a determining unit (34) configured to determine whether the gaze point is present in each of the object determination region (A1, A2) and the entire determination region (A3) based on the detected position of the gaze point;
   an arithmetic unit (35) configured to calculate an object gaze rate indicating a rate at which the gaze point is present in the object determination region (A1, A2) in each of the tasks, and an entire gaze rate indicating a rate at which the gaze point is present in the entire determination region (A3) throughout all the tasks based on a determination result of the determining unit (34); and
   an assessing unit (36) configured to calculate a score based on the object gaze rate and the entire gaze rate calculated by the arithmetic unit (35), and acquire assessment data based on the calculated score.

2. The assessment device (100) according to claim 1, wherein the assessing unit (36) is configured to assign a heavier weight to the entire gaze rate than the object gaze rate, to calculate the score.

3. The assessment device (100) according to claim 1 or 2, further comprising a storage unit (38) configured to store reference data of the object gaze rate and the entire gaze rate, wherein
   the assessing unit (36) is configured to calculate a correlation coefficient indicating a strength of correlation between the reference data stored in the storage unit (38) and the object gaze rate and the entire gaze rate calculated by the arithmetic unit (35), and assign weights to the object gaze rate and the entire gaze rate based on the calculated correlation coefficient, to calculate the score.

4. An assessment program that causes a computer to execute the processes of:

   detecting a position of a gaze point of a subject;
   displaying a plurality of unit images including at least one of an object having specificity (M1) and an object having sensitivity (M2) on a display unit (11) in different tasks;
   setting an object determination region (A1, A2) corresponding to each of the objects and an entire determination region (A3) corresponding to an entirety of the display unit (11);
   determining whether the gaze point is present in each of the object determination region (A1, A2) and the entire determination region (A3) based on the detected position of the gaze point;
   calculating an object gaze rate indicating a rate at which the gaze point is present in the object determination region (A1, A2) in each of the tasks, and an entire gaze rate indicating a rate at which the gaze point is present in the entire determination region (A3) throughout all the tasks based on a determination result; and
   calculating a score based on the object gaze rate and the entire gaze rate calculated, to acquire assessment data based on the calculated score.

**Patentansprüche**

1. Beurteilungsvorrichtung (100), umfassend:

eine Anzeigeeinheit (11);
eine Blickpunkterfassungseinheit (32), die konfiguriert ist, um eine Position eines Blickpunkts eines Subjekts zu erfassen;
eine Anzeigesteuereinheit (31), die konfiguriert ist, um eine Vielzahl von Einheitsbildern, die mindestens eines von einem Objekt mit Spezifität (M1) und einem Objekt mit Empfindlichkeit (M2) umfassen, auf der Anzeigeeinheit (11) in verschiedenen Aufgaben anzuzeigen;
eine Bereichseinstelleinheit (33), die konfiguriert ist, um für jede der Aufgaben einen Objektbestimmungsbereich (A1, A2), der jedem der Objekte entspricht, und einen gesamten Bestimmungsbereich (A3), der einer Gesamtheit der Anzeigeeinheit (11) entspricht, einzustellen;
eine Bestimmungseinheit (34), die konfiguriert ist, um zu bestimmen, ob der Blickpunkt in jedem von dem Objektbestimmungsbereich (A1, A2) und dem gesamten Bestimmungsbereich (A3) vorhanden ist, basierend auf der erfassten Position des Blickpunkts;
eine Arithmetikeinheit (35), die konfiguriert ist, um eine Objektblickrate, die eine Rate angibt, mit der der Blickpunkt in dem Objektbestimmungsbereich (A1, A2) in jeder der Aufgaben vorhanden ist, und eine Gesamtblickrate, die eine Rate angibt, mit der der Blickpunkt in dem gesamten Bestimmungsbereich (A3) in allen Aufgaben vorhanden ist, basierend auf einem Bestimmungsergebnis der Bestimmungseinheit (34) zu berechnen; und
eine Beurteilungseinheit (36), die konfiguriert ist, um einen Wert zu berechnen, basierend auf der Objektblickrate und der Gesamtblickrate, die durch die Arithmetikeinheit (35) berechnet werden, und Beurteilungsdaten basierend auf dem berechneten Wert zu erfassen.

2. Beurteilungsvorrichtung (100) nach Anspruch 1, wobei die Beurteilungseinheit (36) konfiguriert ist, um der Gesamtblickrate ein schwereres Gewicht als der Objektblickrate zuzuordnen, um den Wert zu berechnen.

3. Beurteilungsvorrichtung (100) nach Anspruch 1 oder 2, ferner umfassend eine Speichereinheit (38), die konfiguriert ist, um Referenzdaten der Objektblickrate und der Gesamtblickrate zu speichern, wobei
die Beurteilungseinheit (36) konfiguriert ist, um einen Korrelationskoeffizienten zu berechnen, der eine Stärke der Korrelation zwischen den Referenzdaten, die in der Speichereinheit (38) gespeichert sind, und der Objektblickrate und der Gesamtblickrate, die durch die Arithmetikeinheit (35) berechnet werden, angibt, und der Objektblickrate und der Gesamtblickrate Gewichte zuzuordnen, basierend auf dem berechneten Korrelationskoeffizienten, um den Wert zu berechnen.

4. Beurteilungsprogramm, das einen Computer dazu veranlasst, die folgenden Prozesse auszuführen:

Erfassen einer Position eines Blickpunkts eines Subjekts;
Anzeigen einer Vielzahl von Einheitsbildern, die mindestens eines von einem Objekt mit Spezifität (M1) und einem Objekt mit Empfindlichkeit (M2) umfassen, auf einer Anzeigeeinheit (11) in verschiedenen Aufgaben;
Einstellen eines Objektbestimmungsbereichs (A1, A2), der jedem der Objekte entspricht, und eines gesamten Bestimmungsbereichs (A3), der einer Gesamtheit der Anzeigeeinheit (11) entspricht;
Bestimmen, ob der Blickpunkt in jedem von dem Objektbestimmungsbereich (A1, A2) und dem gesamten Bestimmungsbereich (A3) vorhanden ist, basierend auf der erfassten Position des Blickpunkts;
Berechnen einer Objektblickrate, die eine Rate angibt, mit der der Blickpunkt in dem Objektbestimmungsbereich (A1, A2) in jeder der Aufgaben vorhanden ist, und einer Gesamtblickrate, die eine Rate angibt, mit der der Blickpunkt in dem gesamten Bestimmungsbereich (A3) in allen Aufgaben vorhanden ist, basierend auf einem Bestimmungsergebnis; und
Berechnen eines Werts basierend auf der Objektblickrate und der berechneten Gesamtblickrate, um Beurteilungsdaten zu erfassen, basierend auf dem berechneten Wert.

**Revendications**

1. Dispositif d'évaluation (100) comprenant :

une unité d'affichage (11) ;

une unité de détection de point de regard (32) configurée pour détecter une position d'un point de regard d'un sujet ;

une unité de commande d'affichage (31) configurée pour afficher une pluralité d'images unitaires comprenant au moins l'un d'un objet ayant une spécificité (M1) et d'un objet ayant une sensibilité (M2) sur l'unité d'affichage (11) dans différentes tâches ;

une unité de définition de région (33) configurée pour définir, pour chacune des tâches, une région de détermination d'objet (A1, A2) correspondant à chacun des objets et une région de détermination entière (A3) correspondant à une totalité de l'unité d'affichage (11) ;

une unité de détermination (34) configurée pour déterminer si le point de regard est présent dans chacune de la région de détermination d'objet (A1, A2) et de la région de détermination entière (A3) sur la base de la position détectée du point de regard ;

une unité arithmétique (35) configurée pour calculer une fréquence de regard d'objet indiquant une fréquence à laquelle le point de regard est présent dans la région de détermination d'objet (A1, A2) dans chacune des tâches, et une fréquence de regard entière indiquant une fréquence à laquelle le point de regard est présent dans la région de détermination entière (A3) dans toutes les tâches sur la base d'un résultat de détermination de l'unité de détermination (34) ; et

une unité d'évaluation (36) configurée pour calculer un score sur la base de la fréquence de regard d'objet et de la fréquence de regard entière calculée par l'unité arithmétique (35), et acquérir des données d'évaluation sur la base du score calculé.

2. Dispositif d'évaluation (100) selon la revendication 1, dans lequel l'unité d'évaluation (36) est configurée pour attribuer un poids plus lourd à la fréquence de regard entière que la fréquence de regard d'objet, pour calculer le score.

3. Dispositif d'évaluation (100) selon la revendication 1 ou 2, comprenant en outre une unité de stockage (38) configurée pour stocker des données de référence de la fréquence de regard d'objet et de la fréquence de regard entière, dans lequel

l'unité d'évaluation (36) est configurée pour calculer un coefficient de corrélation indiquant une force de corrélation entre les données de référence stockées dans l'unité de stockage (38) et la fréquence de regard d'objet et la fréquence de regard entière calculée par l'unité arithmétique (35), et attribuer des poids à la fréquence de regard d'objet et à la fréquence de regard entière sur la base du coefficient de corrélation calculé, pour calculer le score.

4. Programme d'évaluation qui amène un ordinateur à exécuter les processus consistant à :

détecter une position d'un point de regard d'un sujet ;

afficher une pluralité d'images unitaires comprenant au moins l'un d'un objet ayant une spécificité (M1) et d'un objet ayant une sensibilité (M2) sur une unité d'affichage (11) dans différentes tâches ;

définir une région de détermination d'objet (A1, A2) correspondant à chacun des objets et une région de détermination entière (A3) correspondant à une totalité de l'unité d'affichage (11) ;

déterminer si le point de regard est présent dans chacune de la région de détermination d'objet (A1, A2) et de la région de détermination entière (A3) sur la base de la position détectée du point de regard ;

calculer une fréquence de regard d'objet indiquant une fréquence à laquelle le point de regard est présent dans la région de détermination d'objet (A1, A2) dans chacune des tâches, et une fréquence de regard entière indiquant une fréquence à laquelle le point de regard est présent dans la région de détermination entière (A3) dans toutes les tâches sur la base d'un résultat de détermination ; et

calculer un score sur la base de la fréquence de regard d'objet et de la fréquence de regard entière calculée, pour acquérir des données d'évaluation sur la base du score calculé.

# FIG.1

# FIG.2

100

| 30 | 60 |
|---|---|

**COMPUTER SYSTEM**

31
DISPLAY CONTROL UNIT

32
GAZE-POINT DETECTING UNIT

33
REGION DETECTING UNIT

34
DETERMINING UNIT

35
ARITHMETIC UNIT

36
ASSESSING UNIT

37
INPUT/OUTPUT CONTROL UNIT

38
STORAGE UNIT

INPUT/ OUTPUT INTERFACE DEVICE

10
DISPLAY DEVICE

20
IMAGE ACQUIRING DEVICE

40
OUTPUT DEVICE

50
INPUT DEVICE

# FIG.3

# FIG.4

START

DISPLAY UNIT IMAGE — S101

SET DETERMINATION REGION — S102

DETECT GAZE POINT — S103

OUTPUT DETERMINATION DATA — S104

CALCULATE PRESENCE TIME — S105

HAS ONE TASK BEEN COMPLETED? — S106

NO

YES

HAVE ALL TASKS BEEN COMPLETED? — S107

YES

NO

DISPLAY UNIT IMAGE OF NEXT TASK — S108

CALCULATE OBJECT GAZE RATE AND ENTIRE GAZE RATE — S109

CALCULATE ASSESSMENT SCORE — S110

CALCULATE ASSESSMENT DATA — S111

END

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2017099513 A
- US 2017112424 A
- US 2017188930 A
- US 2017027805 A
- US 2021290133 A
- US 2020383626 A